# EUROPEAN PATENT SPECIFICATION

(11) **EP 3 483 257 B1**
(45) Date of publication and mention of the grant of the patent: **29.09.2021**
(21) Application number: 17892107.8
(22) Date of filing: 10.08.2017
(51) Int. Cl.: C12N 1/20, C12P 7/24, C12R 1/37

(54) **METHOD FOR PRODUCING BENZALDEHYDE USING MICROORGANISM**
VERFAHREN ZUR HERSTELLUNG VON BENZALDEHYD UNTER VERWENDUNG VON MIKROORGANISMEN
PROCÉDÉ DE PRODUCTION DE BENZALDÉHYDE METTANT EN OEUVRE UN MICRO-ORGANISME

(30) Priority: 20.01.2017 CN 201710051824
(43) Date of publication of application: 15.05.2019
(73) Proprietor: Xiamen Oamic Biotechnology Co., Ltd., Xiamen City, Fujian 361000 (CN)
(72) Inventor: XING, Chenguang, Xiamen Fujian 361000 (CN); ZHAO, Xijing, Xiamen Fujian 361000 (CN); HUANG, Zhiqiang, Xiamen Fujian 361000 (CN); LIU, Wei, Xiamen Fujian 361000 (CN); GONG, Hongcan, Xiamen Fujian 361000 (CN)
(74) Representative: Verscht, Thomas Kurt Albert
(86) International application number: PCT/CN2017/096860
(87) International publication number: WO 2018/133395

(56) References cited:
- EP-A1- 0 193 257
- CN-A- 101 054 365
- CN-A- 101 084 313
- CN-A- 101 985 414
- KR-A- 20110 067 308
- CASEY J ET AL: "Microbial routes to aromatic aldehydes", ENZYME AND MICROBIAL TECHNOLOGY, STONEHAM, MA, US, vol. 14, no. 9, 1 September 1992 (1992-09-01), pages 739-747, XP023679685, ISSN: 0141-0229, DOI: 10.1016/0141-0229(92)90114-4 [retrieved on 1992-09-01]
- MASJA N. NIEROP GROOT ET AL: "Conversion of Phenylalanine to Benzaldehyde Initiated by an Aminotransferase in Lactobacillus plantarum", APPLIED AND ENVIRONMENTAL MICROBIOLOGY, vol. 64, no. 8, 1 January 1998 (1998-01-01), pages 3009-3013, XP55655995, US ISSN: 0099-2240, DOI: 10.1128/AEM.64.8.3009-3013.1998
- TRIVEDI, M.K.: 'Phenotyping and Genotyping Characterization of Proteus vu- lgaris After Biofield Treatment' INTERNATIONAL JOURNAL OF GENETICS AND GENOMICS vol. 3, no. 6, 2015, pages 66 - 73, XP055505493

## Description

### Technical Field

The invention relates to the production of benzaldehyde, in particular to a method for producing benzaldehyde by a microorganism.

### Background of Invention

Benzaldehyde is the simplest aromatic aldehyde, commonly known as bitter almond oil, also known as benzoic aldehyde, the molecular formula of which is C₇H₆O, is a colorless to light yellow volatile liquid. It has a bitter almond flavor and is aromatic when burned. Benzaldehyde is ubiquitous in plants, especially in Rosaceae plants, mainly in the form of glycosides in the bark, leaves or seeds of plants, such as amygdalin in bitter almonds. Benzaldehyde is an important organic chemical raw material. It is mainly used for the production of lauraldehyde, lauric acid, phenylacetaldehyde and benzyl benzoate. It is also an important intermediate for dyes, spices and pharmaceuticals.

### 1 physical properties

Benzaldehyde, with a molecular formula of C₇H₆O, is slightly soluble in water and can be miscible in ethanol, ethyl ether, benzene and chloroform. The pure product is a colorless liquid, the industrial product is a colorless to pale yellow liquid, has the bitter almond odor, with the melting point of 28 °C and boiling point of 178.1 °C. Saturated vapor pressure (kPa) is 0.13(at 26 °C).

### 2, Application

(1). It is important chemical raw materials, used in the production of lauric aldehyde, lauric acid, phenylacetaldehyde and benzyl benzoate, etc., also used as perfumes.

(2). It can be used as a special top fragrance, trace amount of which can be used in flower formula, such as lilac, brandy, jasmine, violet, acacia, sunflower, sweet bean flower, plum flower, orange flower, etc. It is also available in soap. It can also be used as edible spices in almonds, berries, cream, cherries, coconuts, apricots, peaches, walnuts, plums, vanilla beans, spices and other flavors. It is also used in liquor flavors such as rum, brandy and so on.

(3). Intermediates of pharmaceuticals, dyes and fragrances. It is used for production of m-oxybenzaldehyde, lauric acid, lauric aldehyde, green, benzyl benzoate, benzylidene aniline, benzylidene acetone, etc. It is used to blend soap essence, food flavor, etc.

Benzaldehyde can be produced from phenylalanine by the bacterium Lactobacillus plantarum (Nierop Groot M. et al., Applied and Environmental Microbiology, 1998, 64(8): 3009-3013). The species Proteus vulgaris is able to convert amino acids into organic acids, such as phenylalanine into phenyl lactic acid (KR 2011 0067308). It can also transform phenylalanine into phenylpyruvic acid, which can then be converted to benzaldehyde using a mild base (Casey J. et al., Enzyme and Microbial Technology, 1992, 14(9): 739-747).

### Summary of the Invention

The main purpose of the present invention is to provide a strain of *Proteus vulgaris Hauser* OMK-9 with benzaldehyde production capacity. The preservation number of the strain in the China Center for Type Culture Collection (CCTCC) is CCTCC NO: M2016671. The place of preservation is Wuhan University, Wuhan City. The preservation time is November 23, 2016.

Another purpose of the invention is to provide the application of the said strain in the production of benzaldehyde.

The further purpose of the present invention is to provide a method for producing benzaldehyde, which adopts the aforementioned strain.

The invention provides a bacterial strain with benzaldehyde production capacity, and uses the strain to ferment to produce benzaldehyde via fermentation. The fermentation method produces benzaldehyde by using natural raw materials such as saccharide, amino acid, etc. and generates the targer products through a microbial metabolism. The method is a production method which is with low temperature and low pressure, relatively safe, simple operation and less pollution, and is an environmentally friendly production method. The product of the invention has a higher output, and is a production method with industrialization prospect.

### Brief Description of the Drawings

Fig. 1 is a liquid chromatogram of L-phenylalanine standard in embodiment 1.
Fig. 2 is a liquid chromatogram of phenylacetic acid standard in embodiment 1.
Fig. 3 is a liquid chromatogram of benzoic acid standard in embodiment 1.
Fig. 4 is a liquid chromatogram of benzaldehyde standard in embodiment 1.
Fig. 5 is a liquid chromatogram of benzaldehyde fermentation liquid in embodiment 1.

### Detailed Description

### The strain

A strain of *Proteus vulgaris Hauser* OMK-9 with benzaldehyde production capacity was isolated from the soil of a refuse disposal station. The strain was preserved in China Center for Type Culture Collection (CCTCC) on November 23, 2016 under CCTCC NO: M2016671.

The strain: The strain can utilize glucose and sucrose. The 18S rRNA and 26S rRNA sequences of the strain are 99% similar to the sequences of known Bacillus standard strains. The strain is identified as Bacillus. It is gram-negative, with no sporulation, active movement, small roundness at both ends.

The activation of the strain, the cultivation of the seed and the fermentation can be carried out in the following ways:

### 1) the activation the strain:

under sterile conditions, a full inoculation loop of liquid taken from the glycerol stock is uniformly spread on the solid slant medium, and cultured in a biochemical incubator at 25-30 °C for 24-48 hours;

### 2) the cultivation of the seed:

under sterile condition, a well-grown strain is taken from the solid slant medium with a sterile inoculation loop and used to inoculate the sterile seed culture medium, the initial pH of the seed culture medium is 5-8, and the strain is cultured to the logarithmic growth phase at the temperature of 28-35 °C and the rotation speed of 150-500 rpm;

the seed culture medium is, calculated by the mass ratio, formulated as follows: 1.0-3.5% sugar raw materials, 0.1-1.0% inorganic salts and 0.1-1.0% yeast extract powder;

### 3) the fermentation:

the seed liquid cultured to the logarithmic growth phase is used to inoculate fermentation medium at a volume ratio of 5-20% under sterile conditions;

the initial pH of the fermentation medium is 3.0-10.0. at the temperature of 30-40 °C and the rotation speed of 200-500 rpm, under ventilation conditions, the inoculated fermentation medium is fermented for 7-20 h, after which the ventilation is reduced and the fermentation medium is continued to be fermented for 50-100 h.

The fermentation medium by mass ratio consists of 2.0-5.0% sugar raw materials, 0.1-1.0% inorganic salts, 1.0-2.5% yeast extract powder and 1.0-3.0% phenylalanine.

The solid slant medium is preferably a broth medium.

The said sugar raw materials include at least one of glucose, sucrose and starch.

The inorganic salts include potassium salts or sodium salts.

The initial pH of the fermentation medium of step 3) is preferably 5.0-9.0.

### Embodiment 1

### The cultivation of the seed

Under sterile conditions, a well-grown strain culture from a solid slant medium was used to inoculate a sterile seed culture medium. The initial pH of the seed culture medium was 6, and the strain were cultured to the logarithmic growth phase at the temperature of 28 °C and the rotation speed of 150 rpm.

The seed culture medium was formulated by mass ratio(%) as follows: 1.0 Glucose, 0.5 NaCl and 0.1 Yeast extract powder.

### The fermentation

the seed liquid cultured to the logarithmic growth phase is used to inoculate the fermentation medium at a volume ratio of 10% under sterile conditions; The initial pH of the fermentation medium was 5.0. The inoculated fermentation medium was fermented for 15 h at 40 °C, at the rotation speed of 200 rpm and the ventilation rate of 1:0.2. Then the ventilation was reduced to 1:0.02 and the fermentation medium is continued to be fermented for 55 h. The fermentation was formulated as follows (%): 5.0 Glucose, 0.5 NaCl, 1.0 Yeast extract powder and 2.0 Phenylalanine.
Analysis of fermentation liquid:High performance liquid chromatography (HPLC)
Column type: COSMOSIL; 5C18-AR-II; 4.6ID*250MM
Column temperature: 30 °C
Mobile phase: The ratio of methanol to water (0.1% phosphoric acid) is 55:45
Current Speed: 0.8 mL/min, UV: 216 nm.

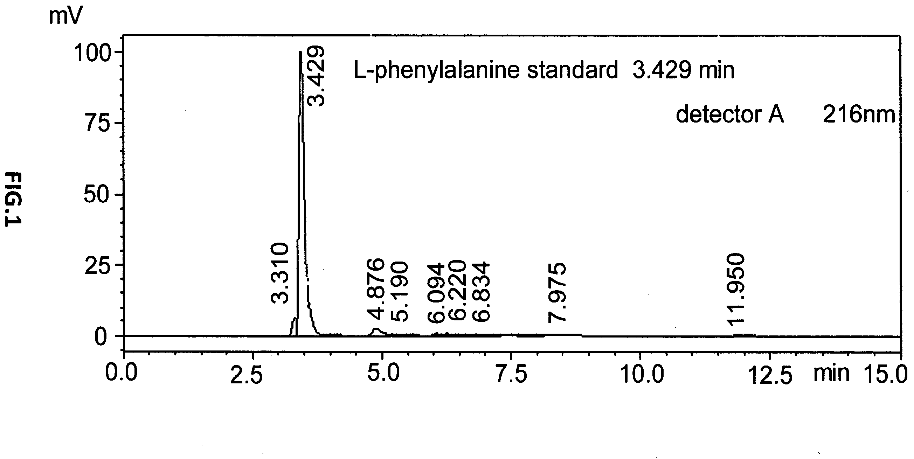

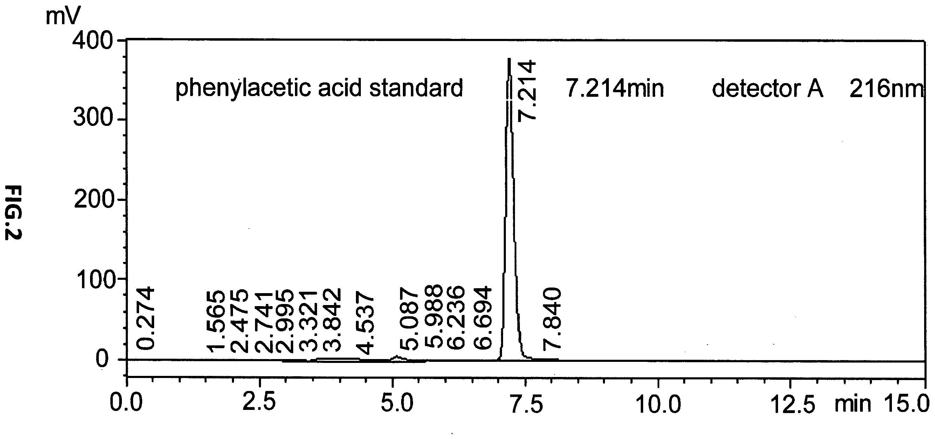

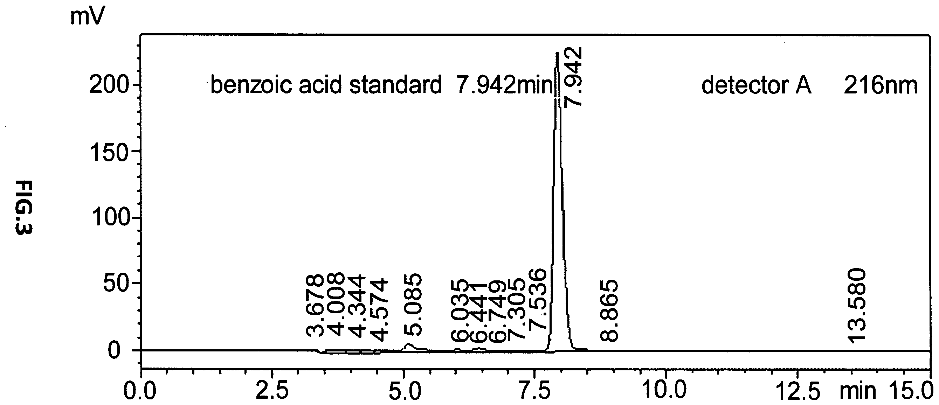

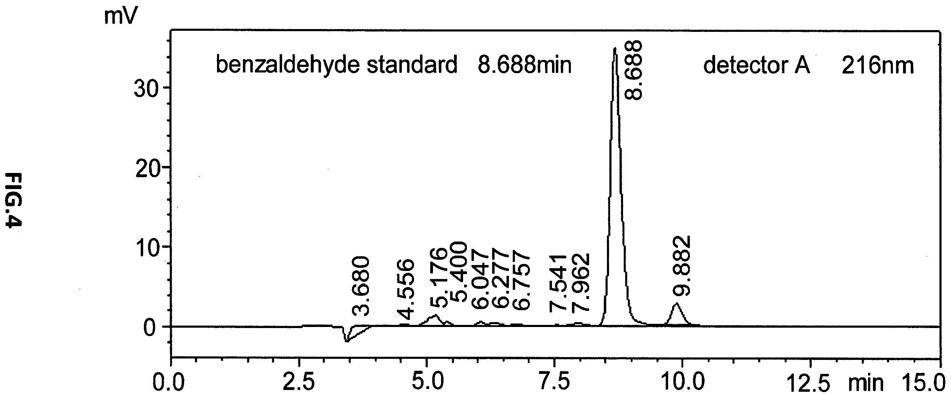

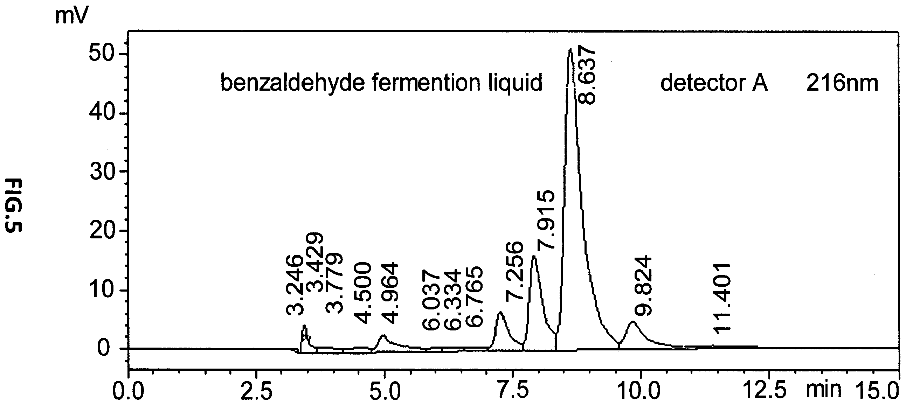

The results are shown in Figures 1 to 5. The results show that the fermentation liquid contains benzaldehyde, benzoic acid, phenylacetic acid and other by-products.

### Embodiment 2

### The cultivation of the seed

Under sterile conditions, from the solid slant medium, a well-grown culture was taken with a sterile inoculation loop and used to inoculate the sterile seed culture medium. The initial pH of the seed culture medium was 5, and the strain was cultured to the logarithmic growth phase at the temperature of 30 °C and the rotation speed of 500 rpm.

The seed medium formula was as follows (%): 3.5 Sucrose, 0.5 KCI and 1.0 Yeast extract powder.

### The fermentation

the seed liquid cultured to the logarithmic growth phase was used to inoculate the fermentation medium at a volume ratio of 10% under sterile conditions; The initial pH of the fermentation medium was 9.0. The inoculated fermentation medium was fermented for 7 hours at 35 °C, 300 rpm rotation speed and 1:0.2 ventilation rate. Then the ventilation was reduced to 1:0.02 and the fermentation medium was continued to be fermented for 73 h.

The fermentation formula is as follows (%): 4 Sucrose, 0.5 KCI, 2.5 Yeast extract powder and 1.5 Phenylalanine.

### Embodiment 3

### The cultivation of the seed

Under sterile conditions, from the solid slant medium, a well-grown culture was taken with a sterile inoculation loop and used to inoculate the sterile seed culture medium. The initial pH of the seed culture medium was 7, and the strain was cultured to the logarithmic growth phase at the temperature of 28 °C and the rotation speed of 500 rpm.

The seed culture medium formula was as follows (%): 3.5 Starch, 0.5 NaCl and 1.0 Yeast extract powder.

### The fermentation

The seed liquid, which was cultured to the logarithmic growth phase, was used to inoculate the fermentation medium at a 15% volume ratio under sterile conditions. The initial pH of the fermentation medium was 7.0, and the inoculated fermentation medium was fermented for 10 hours at 35 °C, 400 rpm rotation speed and 1:0.2 ventilation rate. Then the ventilation was reduced to 1:0.02 and the fermentation medium was continued to be fermented for 65 h. The fermentation formula was as follows (%): 3.5 Starch, 0.5 NaCl, 1.0 Yeast extract powder and 3.0 Phenylalanine.

### Embodiment 4

The production of natural benzaldehyde (oscillating fermentation of triangular bottles)

the seed culture medium was prepared as follows: (g/L): 30 Glucose, 5 potassium dihydrogen, 2.5 sodium chloride and 10 yeast extract powder. The solvent used was water, and the initial pH was 7.0. The seed culture medium was sterilized at 121 °C for 30 min.

The fermentation medium was prepared as follows: (g/L): 30 glucose, 5 potassium dihydrogen phosphate, 8 yeast extract powder and 10.0 phenylalanine. The solvent was water, and the initial pH was 7.0. The fermentation medium was sterilized at 121 °C for 30 min.

the preparation of the seed: Under sterile conditions, the low temperature glycerol tube OMK-9 strain was transferred to a fresh, sterile solid media plate and the inoculated solid media plate was incubated at 28 °C for 2 days. A single colony was picked and used to inoculated into a 500 mL flask for cultivating the seed .The volume of the seed culture medium in the bottle was 50 mL. The shaker was rotated at 180 rpm and cultured at 30 °C for 24 hours to obtain the seed liquid.

The fermentation of natural benzaldehyde. The 50 mL fermentation medium was poured into a 500 mL sterile triangular flask, and then the 7.5 mL seed liquid was inoculated for fermentation. The fermentation temperature was 30 °C and the rotation speed of the shaker was 200 rpm. After shaking the fermentation flask for 20 h, the rotation speed of shaker was reduced to 80 rpm, and the fermentation medium was continued to be fermented for 80 h. Finally the concentration of natural benzaldehyde in fermentation liquid was determined by HPLC as 2.5 g/L.

Embodiment 5 The production of natural benzaldehyde (bioreactor fermentation)

The seed culture medium and the fermentation medium were prepared in the same manner as embodiment 4.

the preparation of the seed :Under sterile conditions, the low temperature glycerol tube OMK-9 strain was transferred to a fresh, sterile solid plate and activated at 28 °C for 2 days. A single colony was picked and used to inoculate into a 3 L stainless steel tank containing 1.8 L seed culture medium for cultivating the seed. The shaker was rotated at 300 rpm and the inoculated seed culture medium was cultured at 30 °C for 24 hours to obtain a seed liquid.

The fermentation of natural benzaldehyde. 10.2 L of the fermentation medium was put into a 20 L fermentor, which was sterilized at 121 °C for 30 min, and then the 1.8 L seed liquid was inoculated into the 20 L fermentor for fermentation. The fermentation temperature was 30 °C, and the rotation speed was 400 rpm. The ventilation ratio was 1: 0.2, and the inoculated fermentation medium was fermented for 10 h. After 10 h, the ventilation was reduced to 1:0.02 and the fermentation medium was continued to be fermented for 70 h. At the end of fermentation, the concentration of natural benzaldehyde in the fermentation medium was determined by HPLC method. The concentration was 5 g/L.

### Industrial Applicability

The invention provided a method with low temperature, low pressure, relatively safe, simple operation and less pollution.

## Claims

1. A strain of *Proteus vulgaris Hauser* OMK-9, which has the ability to produce benzaldehyde, wherein: The strain is stored in China Center for Type Culture Collection (CCTCC) with CCTCC NO: M2016671.

2. The application of the strain according to claim 1 in the production of benzaldehyde.

3. A method for producing benzaldehyde using the strain according to claim 1.

4. The method for producing benzaldehyde according to claim 3, wherein the method comprises the following steps:
1) the activation of the strain:
2) the cultivation of the seed:
in the seed culture medium, the strain is cultured to logarithmic growth phase; the initial pH of the seed culture medium is 5-8, and the seed culture medium by mass ratio is composed of 1.0-3.5% sugar raw materials, 0.1-1.0% inorganic salts and 0.1-1.0% yeast extract powder;
3) the fermentation:
in the fermentation medium, the fermentation medium has an initial pH of 3.0-10.0, and the fermentation medium by mass ratio comprises 2.0-5.0% sugar raw materials, 0.1-1.0% inorganic salts, 1.0-2.5% yeast extract powder and 1.0-3,0% phenylalanine.

5. The method for producing benzaldehyde according to claim 4, wherein the method comprises the following steps:
1) the activation of the strain: under sterile conditions, a full inoculation loop of liquid, which carries the strain from its glycerol stock, is uniformly spread on a solid slant medium, and after spreading, the medium is cultured in a biochemical incubator at 25-30 °C for 24-48 hours;
2) the cultivation of the seed:
the inoculated seed medium is incubated cultured at 28-35 °C and the rotation speed of 150-500 rpm to the logarithmic growth phase.
3) the fermentation steps comprise: the seed liquid culture of the logarithmic growth phase is used to inoculate the fermentation medium at a volume ratio of 5-20% under sterile conditions;
the fermentation medium is fermented at 30-40 °C and a rotation speed of 200-500 rpm, under ventilation conditions for 7-20 h; after which, the ventilation is reduced and the fermentation medium is continued to be fermented for 50-100 h.

6. The method for producing benzaldehyde according to claim 5, wherein, the solid slant medium mentioned in step 1) is a broth medium.

7. The method for producing benzaldehyde according to claim 4, wherein, the sugary raw material mentioned in step 2) and 3) comprises at least one of glucose, sucrose, and starch.

8. The method for producing benzaldehyde according to claim 4, wherein, the inorganic salts mentioned in step 2) and 3) comprise potassium salts or sodium salts.

9. The method for producing benzaldehyde according to claim 4 or 5, wherein, the initial pH of the fermentation medium in step 3) is 5.0-9.0.

## Patentansprüche

1. Ein Stamm von *Proteus vulgaris Hauser* OMK-9, welcher die Fähigkeit hat, Benzaldehyd herzustellen, wobei: der Stamm im China Center for Type Culture Collection (CCTCC) mit CCTCC NR: M2016671 aufbewahrt wird.

2. Die Anwendung des Stammes nach Anspruch 1 bei der Herstellung von Benzaldehyd.

3. Ein Verfahren zur Herstellung von Benzaldehyd unter Verwendung des Stammes nach Anspruch 1.

4. Ein Verfahren zur Herstellung von Benzaldehyd nach Anspruch 3, wobei das Verfahren die folgenden Schritte aufweist:
1) die Aktivierung des Stammes:
2) die Kultivierung des Keims:
in dem Keimkulturmedium, der Stamm wird bis zur logarithmischen Wachstumsphase kultiviert; der anfängliche pH des Keimkulturmediums ist 5-8, und das Keimkulturmedium ist nach Massenverhältnis aus 1,0-3,5% Zuckerrohstoffen, 0,1-1,0% anorganischen Salzen und 0,1-1,0% Hefeextraktpulver zusammengesetzt;
3) die Fermentation:
in dem Fermentationsmedium, das Fermentationsmedium hat einen anfänglichen pH von 3,0-10,0, und das Femientationsmediums weist nach Massenverhältnis 2,0-5,0% Zuckerrohstoffe, 0,1-1,0% anorganische Salze, 1,0-2,5% Hefeextraktpulver und 1,0-3,0% Phenylalanin auf.

5. Das Verfahren zur Herstellung von Benzaldehyd nach Anspruch 4, wobei das Verfahren die folgenden Schritte aufweist:
1) die Aktivierung des Stammes: unter sterilen Bedingungen wird eine volle Impföse von Flüssigkeit, welche den Stamm von seinem Glycerinvorrat trägt, gleichmäßig auf einem festen schrägen Medium verteilt, und nach Verteilung wird das Medium in einem biochemischen Inkubator bei 25-30 °C für 24-48 Stunden kultiviert;
2) die Kultivierung des Keims:
das geimpfte Keimmedium wird inkubiert, und zwar kultiviert bei 28-35 °C und der Rotationsgeschwindigkeit von 150-500 UpM bis zur logarithmischen Wachstumsphase.
3) Die Fermentationsschritte weisen folgendes auf: die Keimflüssigkeitskultur der logarithmischen Wachstumsphase wird verwendet, um das Fermentationsmedium in einem Volumenverhältnis von 5-20% unter sterilen Bedingungen zu impfen;
das Fermentationsmedium wird bei 30-40 °C und einer Rotationsgeschwindigkeit von 200-500 UpM fermentiert, und zwar unter Belüftungsbedingungen für 7-20 Stunden; danach wird die Belüftung reduziert und das Fermentationsmedium wird fortgesetzt, für 50-100 Stunden fermentiert zu werden.

6. Das Verfahren zur Herstellung von Benzaldehyd nach Anspruch 5, wobei das in Schritt 1) genannte feste schräge Medium ein Brühenmedium ist.

7. Das Verfahren zur Herstellung von Benzaldehyd nach Anspruch 4, wobei der in Schritt 2) und 3) genannte Zuckerrohstoff wenigstens eines von Glucose, Saccharose und Stärke aufweist.

8. Das Verfahren zur Herstellung von Benzaldehyd nach Anspruch 4, wobei die in Schritt 2) und 3) genannten anorganischen Salze Kaliumsalze oder Natriumsalze aufweisen.

9. Das Verfahren zur Herstellung von Benzaldehyd nach Anspruch 4 oder 5, wobei der anfängliche pH des Fermentationsmediums in Schritt 3) 5,0-9,0 ist.

## Revendications

1. Souche de *Proteus vulgaris Hauser* OMK-9, qui a la capacité de produire du benzaldéhyde, dans laquelle : la souche est stockée au Center for Type Culture Collection de Chine (CCTCC) avec le numéro CCTCC M2016671.

2. Application de la souche selon la revendication 1 dans la production de benzaldéhyde.

3. Procédé pour produire du benzaldéhyde utilisant la souche selon la revendication 1.

4. Procédé pour produire du benzaldéhyde selon la revendication 3, dans lequel le procédé comprend les étapes suivantes :
1) l'activation de la culture ;
2) la culture de la semence :
dans le milieu de culture de semence, la souche est cultivée jusqu'à la phase de croissance logarithmique ; le pH initial du milieu de culture de semence est de 5 à 8, et le milieu de culture de semence en proportion en masse est composé de 1,0 à 3,5 % de matières premières de sucre, 0,1 à 1,0 % de sels inorganiques et 0,1 à 1,0 % de poudre d'extrait de levure ;
3) la fermentation :
dans le milieu de fermentation, le milieu de fermentation a un pH initial de 3,0 à 10,0, et le milieu de fermentation en proportion en masse comprend 2,0 à 5,0 % de matières premières de sucre, 0,1 à 1,0 % de sels inorganiques, 1,0 à 2,5 % de poudre d'extrait de levure et 1,0 à 3,0 % de phénylalanine.

5. Procédé pour produire du benzaldéhyde selon la revendication 4, dans lequel le procédé comprend les étapes suivantes :
1) l'activation de la souche : dans des conditions stériles, une boucle d'inoculation complète de liquide, qui transporte la souche depuis son stock de glycérol, est uniformément étalée sur un milieu incliné solide, et, après l'étalement, le milieu est cultivé dans un incubateur biochimique à 25-30°C pendant 24 à 48 heures ;
2) la culture de la semence :
le milieu de semence inoculé est incubé par culture à 28-35°C et à une vitesse de rotation de 150 à 500 t/min jusqu'à la phase de croissance logarithmique ;
3) les étapes de fermentation comprennent : la culture du liquide de semence de la phase de croissance logarithmique est utilisée pour inoculer le milieu de fermentation en un rapport en volume de 5 à 20 % dans des conditions stériles ;
le milieu de fermentation est fermenté à 30-40°C et à une vitesse de rotation de 200 à 500 t/min, dans des conditions ventilées pendant 7 à 20 heures ; après quoi la ventilation est réduite et le milieu de fermentation continue d'être fermenté pendant 50 à 100 heures.

6. Procédé pour produire du benzaldéhyde selon la revendication 5, dans lequel le milieu incliné solide mentionné dans l'étape 1) est un milieu de bouillon.

7. Procédé pour produire du benzaldéhyde selon la revendication 4, dans lequel la matière première sucrée mentionnée dans les étapes 2) et 3) comprend au moins l'un parmi le glucose, le saccharose, et l'amidon.

8. Procédé pour produire du benzaldéhyde selon la revendication 4, dans lequel les sels inorganiques mentionnés dans les étapes 2) et 3) comprennent des sels de potassium ou des sels de sodium.

9. Procédé pour produire du benzaldéhyde selon la revendication 4 ou 5, dans lequel le pH initial du milieu de fermentation dans l'étape 3) est de 5,0 à 9,0.
